# EUROPÄISCHE PATENTANMELDUNG

(11) **EP 1 180 382 A1**
(43) Veröffentlichungstag der Anmeldung: **20.02.2002**
(21) Anmeldenummer: 01119446.1
(22) Anmeldetag: 13.08.2001
(51) Int. Cl.: A61M 39/28

(54) **Vorrichtung zum Verschluss eines Dauerkatheters**

(30) Priorität: 17.08.2000 DE 10040337
(71) Anmelder: Hartmann, Peter, 82418 Murnau (DE)
(72) Erfinder: Hartmann, Peter Dipl. Ing., 82418 Murnau (DE); Löchner-Ernst, Dieter Dr. med., 82418 Murnau (DE)
(74) Vertreter: Flosdorff, Jürgen, Dr.

(57) **Zusammenfassung**

Die Vorrichtung zum Verschluß eines Dauerkatheters besteht aus einem sterilen, universell passenden Einmal-Adapter, der eine Anschlußeinrichtung mit einem in den Dauerkatheter einsteckbaren Konusstück und ein aus einem weichelastischen Material bestehendes Schlauchstück aufweist. Auf dem Schlauchstück sitzt eine federbeaufschlagte Klemmeinrichtung, die im Gegensatz zu herkömmlichen Verschlüssen von Dauerkathetern nicht mit Urin in Kontakt gerät. Der Einmal-Katheter wird täglich gewechselt, wodurch Infektionen durch Kontamination vermieden wird. Die Klemmeinrichtung ist äußerst einfach einhändig zu bedienen.

## Beschreibung

Die Erfindung betrifft eine Vorrichtung zum Verschluß eines Dauerkatheters.

Patientinnen oder Patienten, die postoperativ oder aus unterschiedlichen urologischen Gründen, sei es durch direkten Blasenzugang durch die Harnröhre oder operativ durch die Bauchdecke einen Dauerkatheter gelegt bekommen, verschließen diesen bisher mit einer teuren Verschlußeinrichtung, die in das freie Ende des Dauerkatheters eingesteckt ist. Diese im Dauerkatheter angeordneten Verschlüsse haben den Nachteil, daß sie täglich gewechselt und sterilisiert werden sollten, um Infektionen durch Kontamination vorzubeugen. Da die Verschlüsse einen komplizierten Aufbau haben, ist eine vollständige Sterilität dennoch nicht zu gewährleisten. Ein weiterer Nachteil der bekannten Verschlüsse besteht darin, daß sie relativ enge Durchgänge haben, die durch Sediment oder Mikropartikel aus der Blase oder durch kleinere Nieren- oder Blasensteine verstopft werden können. Die bisher bekannten Verschlußvorrichtungen sind zudem wegen ihrer relativ komplizierten Bauweise fehleranfällig und für ältere Patientinnen oder Patienten oftmals schwer zu verstehen und zu bedienen. Wenn sie -trotz richtiger Bedienung durch die Patienten- nicht durchlässig sind, ist die Folge eine übervolle Blase, schlimmstenfalls mit Reflux und Nierenstau.

Der vorliegenden Erfindung liegt die Aufgabe zugrunde, eine kostengünstige Vorrichtung zum Verschluß eines Dauerkatheters anzugeben, bei der weitestgehend Infektionen durch Kontamination und Verstopfung vermieden werden und die leicht handhabbar ist.

Diese Aufgabe wird erfindungsgemäß durch die Merkmale des Patentanspruchs 1 gelöst. Vorteilhafte Ausgestaltungen der Erfindung sind in den Unteransprüchen gekennzeichnet.

Die Erfindung sieht vor, daß die Verschlußeinrichtung einen Adapter enthält, der eine Anschlußeinrichtung für den Dauerkatheter und ein aus einem weichelastischen Material bestehendes Schlauchstück aufweist, und daß dieses Schlauchstück mittels einer Klemmeinrichtung verschließbar ist. Das Schlauchstück besteht vorzugsweise aus Silikon oder einem anderen, ebenso weich-elastischen Material. Die Klemmeinrichtung sitzt außen auf dem Schlauch, so daß sie nicht mit Urin in Kontakt gerät.

Der Adapter ist aufgrund seiner einfachen Bauweise nur mit geringen Kosten verbunden, so daß er als steriler, universell passender Einmal-Adapter täglich gewechselt werden kann. Die außen auf dem Adapter sitzende Klemmeinrichtung ist -da sie nicht mit Urin in Kontakt gerät- hingegen über einen langen Zeitraum verwendbar.

Da das Schlauchstück weich und damit leicht biegsam ist, paßt es sich den Körpergegebenheit an, was vor allem bei Bauchkathetern die bisher durch die Verschlußeinrichtung verursachten Schmerzen vermeidet. Da die Klemmeinrichtung außen auf dem Schlauch sitzt und nicht -wie beim Stand der Technik- in den Abflußquerschnitt eingesteckt ist, gibt sie in der Freigabestellung den gesamten Querschnitt des Schlauchstücks frei, wodurch Verstopfungen sicher vermieden werden können.

In näheren Einzelheiten wird vorgeschlagen, daß die Anschlußeinrichtung, die aus einem harten Kunststoff wie PA6 oder dergleichen besteht, einen Konusabschnitt aufweist, der in den Dauerkatheter dicht einsteckbar ist. Der Konusabschnitt hat eine solche Form, daß er universell in alle marktüblichen Katheter paßt.

An den Konusabschnitt schließt ein kreiszylinderischer Abschnitt an, auf dem das Schlauchstück, das etwa 10 cm lang ist, befestigt ist. Bevorzugt wird das Schlauchstück auf den zylindrischen Stutzen aufgeklebt.

Die Anschlußeinrichtung und das Schlauchstück haben Durchgangskanäle in einer solchen Größe, daß gewährleistet ist, daß es durch eventuelle Sedimentausscheidung, Mikropartikel aus der Blase oder kleinen Nieren- oder Blasensteine nicht zu einer Verstopfung kommen kann.

Die erfindungsgemäße Klemmeinrichtung besteht aus einem Grundkörper, der bevorzugt im wesentlichen eine Kugelform hat und eine mittige Durchgangsbohrung aufweist, durch die das Schlauchstück hindurchführbar ist, und einem Klemmkörper, der eine ebenso geformte Durchgangsbohrung hat und zwischen einer Freigabeposition, in der die beiden Durchgangsbohrungen miteinander fluchten, und einer Klemmposition verschiebbar in dem Grundkörper sitzt, in der die Durchgangsbohrungen so gegeneinander versetzt sind, daß das Schlauchstück dicht zusammengequetscht ist. Dabei wird der Klemmkörper von einer nicht-rostenden Feder in die Klemmposition gedrückt. Die Durchgangsöffnungen haben zweckmäßigerweise eine längliche Form, so daß das hindurchgeführte Schlauchstück in einer Richtung leicht zusammengedrückt ist.

Der Klemmkörper ist unverlierbar in dem Grundkörper angeordnet, wobei z.B. Anschläge den Bewegungsbereich so begrenzen, daß der Klemmkörper sich nicht über die Klemmstellung, in der der Schlauchquerschnitt zugequetscht ist, hinausbewegen kann. Wenn andererseits der Klemmkörper durch Ausübung von Druck gegen die Kraft der Feder in die Freigabeposition gedrückt wird, liegt er am Boden der zur Aufnahme des Klemmkörpers vorgesehenen Bohrung des Grundkörpers an, wenn die beiden Durchgangsöffnungen miteinander fluchten.

Der Klemmkörper hat bevorzugt eine von der Kreisform abweichende Außenkontur, so daß er in der entsprechend geformten Bohrung des Grundkörpers nicht-drehbar geführt ist.

Weiter wird vorgeschlagen, daß der Klemmkörper in der Klemmposition lösbar verriegelbar ist, damit ein versehentliches Öffnen der Ausflußöffnung ausgeschlossen ist. Hierbei kann die Ausbildung so getroffen sein, daß der Klemmkörper bei Erreichen der Klemmposition vorzugsweise um 90° gedreht werden kann, um z.B. in eine leicht lösbare Raststellung zu gelangen. Es kann aber beispielsweise auch ein am Grundkörper geführter Schieber vorgeschoben werden, der in eine Öffnung des Klemmkörpers eingreift und diesen blockiert.

Die Klemmeinrichtung ist auch von älteren Personen oder Behinderten mit eingeschränkter Handgreiffunktion sehr einfach zu bedienen. Durch die Federvorspannung schließt die Klemmeinrichtung selbsttätig und damit absolut sicher, so daß es hier nicht zu Fehlbedienungen kommen kann.

Weitere Einzelheiten der Erfindung ergeben sich aus der nachfolgenden Beschreibung einer bevorzugte Ausführungsform sowie anhand der Zeichnung. Dabei zeigen:

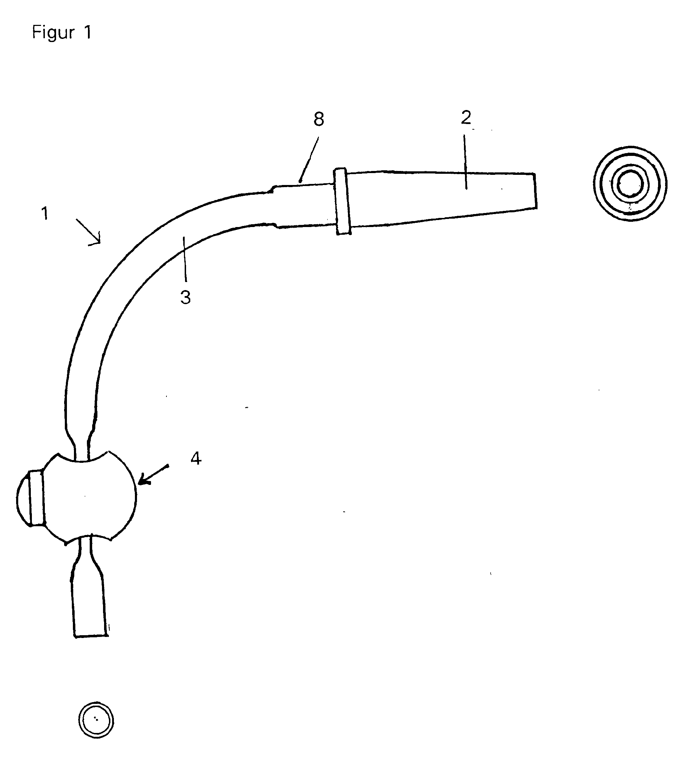

- Fig. 1: eine Seitenansicht der gesamten Verschlußvorrichtung;
- Fig. 2: eine Stirnansicht der Klemmeinrichtung im Klemmzustand;
- Fig. 3: die Klemmeinrichtung gemäß Fig. 2 im Freigabezustand;
- Fig. 4: eine rein schematische Darstellung der Klemmeinrichtung im Freigabezustand;
- Fig. 5: eine Aufsicht auf den Grundkörper der Klemmeinrichtung;
- Fig. 6: eine Aufsicht auf den Klemmkörper der Klemmeinrichtung und
- Fig. 7: einen Längsschnitt durch die Anschlußeinrichtung der Verschlußvorrichtung.

Die Figuren zeigen weitgehend schematisch die Verschlußvorrichtung 1, die aus einer Anschlußeinrichtung 2, einem bevorzugt etwa 10 cm langen weichelastischen Schlauchstück 3 und einer Klemmeinrichtung 4 besteht.

Die Anschlußeinrichtung besteht aus einem harten Kunststoff wie PA6 und enthält einen Konusabschnitt 5, der für alle marktüblichen Katheter paßt, und einen kreiszylindrischen Stutzen 6, der durch einen Ringbund 7 von dem Konusabschnitt 5 getrennt ist. Die Anschlußeinrichtung 2 ist als einteiliges Spritzgußteil hergestellt.

Das Schlauchstück 3 ist mit einem Endabschnitt 8 auf dem zylindrischen Stutzen 6 festgeklebt. Der Durchlaßkanal 9 der Anschlußeinrichtung 2 und der lichte Innenraum des Schlauchstücks 3 haben bevorzugt denselben Durchmesser, der so groß bemessen ist, daß es nicht zu Verstopfungen kommen kann.

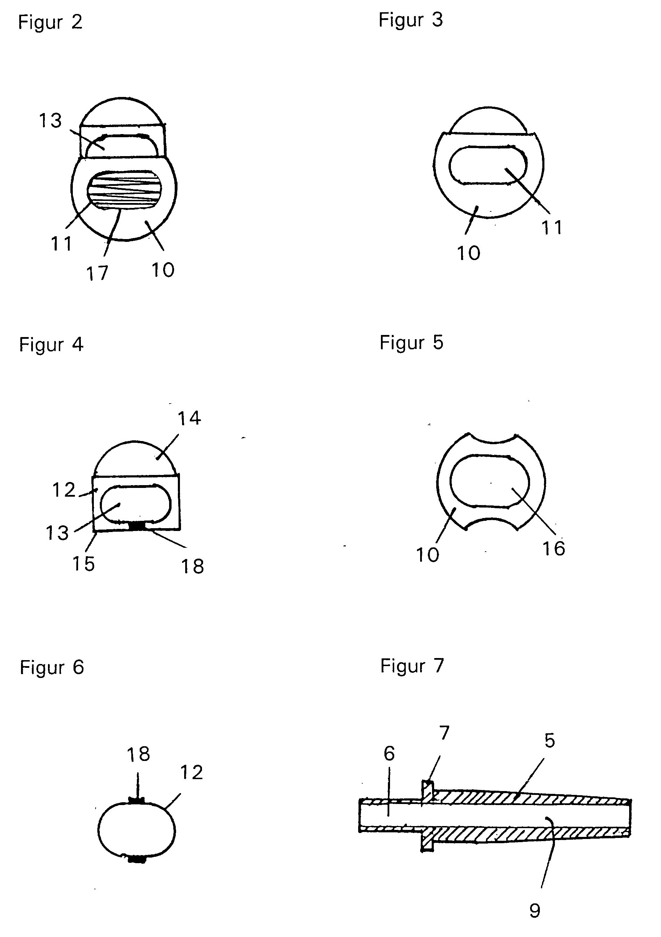

Die Klemmeinrichtung 4 besteht aus einem etwa kugelförmigen Grundkörper 10, der eine etwa mittige Durchgangsöffnung 11 hat. In dem Grundkörper 10 sitzt quer zur Durchgangsöffnung 11 verschiebbar ein Klemmkörper 12, der eine Durchgangsbohrung 13 identischer Form und Größe wie die Durchgangsbohrung 11 besitzt. Der Klemmkörper 12 hat einen gewölbten oberen Betätigungsabschnitt 14, auf den ein Benutzer eine Druckkraft ausübt, wenn er die Klemmeinrichtung 4 in den in Figur 3 dargestellten Freigabezustand versetzen will, in dem die beiden Durchgangsöffnungen 11 und 13 miteinander fluchten. Diese Position ist durch den Boden einer Sackbohrung 16 (Fig. 5) definiert, in der der Klemmkörper in der Darstellung der Figuren 2 bis 4 in vertikaler Richtung geführt ist.

Zwischen dem Boden der Sackbohrung 16 und der Unterseite des Klemmkörpers 12 ist eine nicht-rostende Feder 17 angeordnet, die den Klemmkörper 12 nach oben in den in Fig. 2 dargestellten Klemmzustand drückt, in dem das durch die Durchgangsbohrungen 11 und 13 hindurchgeführte Schlauchstück 3 so zusammengequetscht ist, daß das Schlauchstück absolut dicht verschlossen ist.

In dem Klemmzustand liegen zwei seitlich unterhalb der Durchgangsbohrung 13 angegossene keilförmige Nasen 18 an Anschlägen an, wodurch das Herausfallen bzw. Herausfedern des Klemmkörpers 12 aus der Sackbohrung 16 verhindert wird.

Die Sackbohrung 16 hat in der dargestellten Ausführungsform eine etwa elliptische Grundrißform. Der Klemmkörper 12 hat eine identische Kontur (Fig. 6), die verhindert, daß sich der Klemmkörper 12 in der Sackbohrung 16 dreht.

In der in Fig. 2 dargestellten Klemmstellung kann aber die Sackbohrung 16 kreisförmig verbreitert sein, so daß der Klemmkörper 12 in der Klemmposition in eine Haltestellung gedreht werden kann, in der verhindert ist, daß der Klemmkörper 12 unbeabsichtigt nach unten in die Freigabeposition gedrückt wird.

Der Konusabschnitt 5 kann umlaufende Rillen aufweisen, in denen sich beim Einstecken in den Innenkonus des Katheters evtl. an den Wänden des Innenkonus haftende Feuchtigkeit abstreift und somit ein festerer Sitz erreicht wird.

## Patentansprüche

1. Vorrichtung zum Verschluß eines Dauerkatheters,
**gekennzeichnet durch**
einen Adapter, der eine Anschlußeinrichtung (2) und ein aus einem weichelastischen Material bestehendes Schlauchstück (3) aufweist, und eine Klemmeinrichtung (4) für das Schlauchstück (3).

2. Vorrichtung nach Anspruch 1,
**dadurch gekennzeichnet, daß** die Anschlußeinrichtung (2) einen Konusabschnitt (5) aufweist, der in den Dauerkatheter dicht einsteckbar ist.

3. Vorrichtung nach Anspruch 1 oder 2,
**dadurch gekennzeichnet, daß** die Anschlußeinrichtung (2) einen kreiszylindrischen Abschnitt (6) aufweist, auf dem das Schlauchstück (3) befestigt ist.

4. Vorrichtung nach einem der Ansprüche 1 bis 3,
**dadurch gekennzeichnet, daß** die Anschlußeinrichtung (2) aus einem hartem Kunststoff wie PA6 besteht.

5. Vorrichtung nach einem der Ansprüche 1 bis 4,
**dadurch gekennzeichnet, daß** die Klemmeinrichtung (4) einen Grundkörper (10) aufweist, der eine Durchgangsbohrung (11) hat, durch die das Schlauchstück (3) hindurchführbar ist, und daß in dem Grundkörper (10) ein Klemmkörper (12) angeordnet ist, der eine übereinstimmende Durchgangsbohrung (13) hat und zwischen einer Freigabeposition (Fig. 3) und einer Klemmposition (Fig. 2) verschiebbar ist, in der die Durchgangsbohrungen (11, 13) so gegeneinander versetzt sind, daß das Schlauchstück (3) dicht zusammengequetscht ist.

6. Vorrichtung nach Anspruch 5,
**dadurch gekennzeichnet, daß** der Klemmkörper (12) von einer Feder (17) in die Klemmposition vorgespannt ist.

7. Vorrichtung nach Anspruch 5 oder 6,
**dadurch gekennzeichnet, daß** die Durchgangsöffnungen (11, 13) eine längliche Form haben, so daß das hindurchgeführte Schlauchstück (3) in einer Richtung leicht zusammengedrückt ist.

8. Vorrichtung nach einem der Ansprüche 5 bis 7,
**dadurch gekennzeichnet, daß** der Klemmkörper (12) unverlierbar in dem Grundkörper (10) angeordnet ist.

9. Vorrichtung nach einem der Ansprüche 5 bis 8,
**dadurch gekennzeichnet, daß** der Klemmkörper (12) in der Klemmposition lösbar verriegelbar ist.
